**Europäisches Patentamt**

**European Patent Office**

**Office européen des brevets**

(11) Veröffentlichungsnummer : **0 023 346**
**B1**

(12) **EUROPÄISCHE PATENTSCHRIFT**

(45) Veröffentlichungstag der Patentschrift :
13.07.83

(51) Int. Cl.³ : **C 12 P 13/04**

(21) Anmeldenummer : 80104345.6

(22) Anmeldetag : 24.07.80

(54) **Verfahren zur kontinuierlichen enzymatischen Umwandlung von wasserlöslichen alpha-Ketocarbonsäuren in die entsprechenden Aminosäuren.**

(30) Priorität : 25.07.79 DE 2930070

(43) Veröffentlichungstag der Anmeldung :
04.02.81 Patentblatt 81/05

(45) Bekanntmachung des Hinweises auf die Patenterteilung : 13.07.83 Patentblatt 83/28

(84) Benannte Vertragsstaaten :
BE CH DE FR GB LI NL SE

(56) Entgegenhaltungen :
FR A 2 033 404
FR A 2 217 296
FR A 2 293 489
FR A 2 398 046
US A 3 915 799

APPLIED BIOCHEMISTRY AND MICROBIOLOGY,
Band 14, Nr. 6, Mai 1979, Seiten 706-710, Plenum
Press U.S.A.

Die Akte enthält technische Angaben, die nach dem
Eingang der Anmeldung eingereicht wurden und die
nicht in dieser Patentschrift enthalten sind.

(73) Patentinhaber : Degussa Aktiengesellschaft
Weissfrauenstrasse 9
D-6000 Frankfurt am Main 1 (DE)

Gesellschaft für Biotechnologische Forschung mbH
(GBF)
Mascheroder Weg 1
D-3300 Braunschweig-Stöckheim (DE)

(72) Erfinder : Wandrey, Christian, Prof. Dr. Dipl.-Chem.
Berliner Strasse 23
D-5170 Jülich (DE)
Erfinder : Wichmann, Rolf, Dipl.-Chem.
Merkatorstrasse 13
D-5170 Jülich (DE)
Erfinder : Leuchtenberger, Wolfgang, Dr. Dipl.-Chem.
Geschwister-Scholl-Strasse 1
D-6454 Bruchköbel (DE)
Erfinder : Kula, Maria-Regina, Dr. Dipl.-Chem.
Forstweg 15
D-3340 Wolfenbüttel (DE)
Erfinder : Bückmann, Andreas, Dipl.-Ing.
Kutheweg 4
D-3300 Braunschweig-Stöckheim (DE)

# Verfahren zur kontinuierlichen enzymatischen Umwandlung von wasserlöslichen α-Ketocarbonsäuren in die entsprechenden Aminosäuren

Die Erfindung betrifft ein Verfahren zur kontinuierlichen enzymatischen Umwandlung von wasserlöslichen α-Ketocarbonsäuren in die entsprechenden Aminosäuren in Gegenwart einer substratspezifischen Dehydrogenase, von Ammoniumionen und von durch Bindung an ein wasserlösliches Polymeres im Molekulargewicht vergrössertem Nicotinamid-adenin-dinucleotid (NAD$^+$/NADH) in einem mit einer Ultrafiltrationsmembran ausgestatteten Membranreaktor unter gleichzeitiger Regenierung von NADH aus NAD$^+$ mittels Formiationen in Gegenwart einer Formiatdehydrogenase.

Enzymatische Umsetzungen in Membranreaktoren werden zwar bereits seit längerer Zeit untersucht, aber üblicherweise ohne erzwungene Konvektion des Reaktionsgemisches über die Membran und mit sehr geringen Substratkonzentrationen.

So ist beispielsweise in den Preprints zum « First European Congress on Biotechnology » vom 25. bis 29. September 1978 in Interlaken, DECHEMA, Frankfurt am Main, 1978, 2/44 bis 47 ein Batchversuch zur Umwandlung von Pyruvat in Alanin in Gegenwart von durch Anlagerung an ein Dextran (Molekulargewicht 40 000) im Molekulargewicht vergrößertem NAD$^+$/NADH, von Alanindehydrogenase und von Ammoniumionen unter gleichzeitiger Regenerierung von NADH mittels Formiat in Gegenwart von Formiatdehydrogenase beschrieben.

Aus der FR-A-2 217 296 ist ebenfalls bereits ein diskontinuierlich betriebenes Verfahren zur enzymatischen Umwandlung von α-Ketocarbonsäuren in die entsprechenden α-Aminocarbonsäuren in Gegenwart von nativem NAD$^+$/NADH, einer substratspezifischen Dehydrogenase und von Ammoniumionen unter gleichzeitiger Regenerierung von NADH mittels Formiat in Gegenwart von Formiatdehydrogenase bekannt.

In der FR-A-2 398 046 schließlich ist im Zusammenhang mit der enzymatischen Umwandlung von Dehydrocarnitin in Carnitin die Verwendung von an eine hochmolekulare, lösliche Substanz gebunden vorliegendem NAD$^+$/NADH als Coenzym erwähnt. Nähere Angaben über geeignete hochmolekulare Substanzen finden sich jedoch nicht.

Eine Anwendung solcher Umsetzungen im Produktionsmaßstab scheiterte bisher daran, daß eine kontinuierliche Verfahrensführung nicht möglich war, und daß sich nur geringe Raum-Zeit-Ausbeuten erzielen ließen.

Das erfindungsgemäße Verfahren ist nun dadurch gekennzeichnet, daß man einem Membranreaktor, dessen Membran einen mittleren Porendurchmesser von 1 bis 3 nm aufweist, und der eine Lösung der Formiatdehydrogenase, der substratspezifischen Dehydrogenase und von 0,1 bis 10 mmol/l an ein Polyoxyäthylen mit einem durchschnittlichen Molekulargewicht zwischen 500 und 50 000 gebunden vorliegendem NAD$^+$/NADH enthält, kontinuierlich eine wässrige Lösung von 50 bis 100 % der maximal löslichen Menge, jedoch nicht über 2 000 mmol/l, der umzusetzenden α-Ketocarbonsäure in Form eines wasserlöslichen Salzes als Substrat, einer der Substratmenge etwa äquimolaren Menge an Ammoniumionen, und von 100 bis 6 000 mmol/l eines Formiats zuführt, über die Membran einen Differenzdruck von 0,1 bis 15 bar aufrechterhält und hinter der Membran kontinuierlich einen die gebildete Aminosäure enthaltenden Filtratstrom abführt.

Das erfindungsgemäße Verfahren erlaubt es, wasserlösliche α-Ketocarbonsäuren kontinuierlich und mit hohen Raum-Zeit-Ausbeuten in die entsprechenden Aminosäuren umzuwandeln und ist daher zu einer kostengünstigen Produktion dieser Aminosäuren anwendbar.

Als Reaktionsgefäss wird ein mit einer Ultrafiltrationsmembran ausgestatteter Membranreaktor verwendet, dessen Membran dazu dient, die eingesetzten Enzyme und das für die Umsetzung erforderliche Coenzym im Reaktor zurückzuhalten, aber das niedermolekulare Produkt und das nicht umgesetzte Substrat durchzulassen. Der Membranreaktor kann als sogenannter Flachmembranreaktor ausgebildet sein. Bei diesem Reaktortyp kann es sich beispielsweise um ein flaches zylindrisches Gefäss handeln, auf das ein mittels eines O-Ringes abgedichteter Deckel aufgesetzt ist. Zusammen mit dem O-Ring ist die flächenmässig relativ ausgedehnte flache Membran eingespannt. Der Substratstrom wird durch eine Dosierpumpe dem unterhalb der Membran liegenden Reaktionsraum zugeführt, der zweckmässigerweise mit einer Rühreinrichtung, z. B. einem Magnetrührer, ausgestattet ist. Der das Produkt enthaltende Filtratstrom verlässt den Reaktionsraum durch die Membran und eine zwecks Vermeidung von deren mechanischer Beanspruchung darüber angeordnete, mit Bohrungen versehene Platte und wird aus dem Deckel abgeführt. Ein sogenannter Hohlfaser-Membranreaktor, in dem ein Hohlfaserbündel aus Ultrafiltrationsmembranen, ein sogenanntes Hohlfaser-Modul, an die Stelle der Flachmembran tritt, ist dann vorteilhafter, wenn sich aufgrund der geometrischen Anordnung höhere Reynoldzahlen des Fluids parallel zur Membran und damit geringere Belegungen der Membran mit Enzymproteinen erreichen lassen. Bei diesem Reaktortyp handelt es sich beispielsweise um eine Art von Schlaufenreaktor, der aus einem Reaktionsbehälter, einer Umwälzpumpe und dem Hohlfaser-Modul besteht. Der Substratstrom wird mittels einer Dosierpumpe dem Reaktionsbehälter zugeführt. In diesem wird das Reaktionsgemisch umgepumpt, wobei der Umpumpstrom im Verhältnis zum Substratstrom mindestens etwa 100 : 1 beträgt, um die Belegung der Hohlfasermembranen mit Enzymprotein so gering wie möglich zu halten. Der das Produkt enthaltende Filtratstrom tritt durch die Hohlfasermembranen hindurch und wird hinter diesen gesammelt und abgeführt. Für das erfindungsgemässe Verfahren werden Membranen verwendet, die einen mittleren Porendurchmesser von 1 bis 3 nm aufweisen. Geeignete Materialien für die Membranen sind beispielsweise Acetylcellulosen, Polyamide, Polysulfone oder modifizierte Polyvinylalkohole.

Der Membranreaktor enthält eine Lösung einer Formiatdehydrogenase, einer substratspezifischen Dehydrogenase und von im Molekulargewicht vergrössertem $NAD^+/NADH$. Die Formiatdehydrogenase wird zweckmässig in einer solchen Menge eingesetzt, dass ihre Aktivität mindestens 12 000 μmol/l · Minute beträgt. Nach oben sollte ihre Einsatzmenge zweckmässigerweise so begrenzt werden, dass die Proteinkonzentration maximal etwa 20 g/l beträgt. Die substratspezifische Dehydrogenase wird zweckmässig in einer solchen Menge eingesetzt, dass das Verhältnis der Aktivitäten von Formiatdehydrogenase und substratspezifischer Dehydrogenase zwischen 1 : 1 und 1 : 5 liegt.

Das beim erfindungsgemässen Verfahren als Coenzym erforderliche $NAD^+/NADH$ muss durch Bindung an ein Polyoxyäthylen in seinem Molekulargewicht soweit vergrössert sein, dass es zwar noch wasserlöslich ist, um eine homogene Katalyse zu erlauben, andererseits aber zusammen mit den beiden Enzymen durch die Membran sicher zurückgehalten wird. Zu diesem Zweck wird beispielsweise das Coenzym in seiner oxydierten Form zunächst mit Äthylenimin zum N(1)-Aminoäthylderivat umgesetzt, welches dann seinerseits mit Hilfe der Carbodiimidmethode (vgl. Cuatrecanas, J. Biol. Chem., 245, 3 059 (1970) an ein carboxyliertes Polyäthylenglykol gekuppelt wird, dessen Polyoxyäthylenkette ein durchschnittliches Molekulargewicht zwischen 500 und 50 000, vorzugsweise zwischen 1 500 und 10 000, aufweist. Das erhaltene Kupplungsprodukt wird dann zum entsprechenden NADH-Derivat reduziert, durch eine Dimroth-Umlagerung in das N(6)-Derivat umgewandelt und gegebenenfalls wieder zum entsprechenden $NAD^+$-Derivat oxydiert. Das im Molekulargewicht vergrösserte Coenzym wird in einer solchen Menge eingesetzt, dass die Konzentration an $NAD^+/NADH$ 0,1 bis 10 mmol/l, vorzugsweise 1 bis 7 mmol/l, beträgt.

Dem Membranreaktor wird kontinuierlich eine wässrige Lösung des Substrats, von Ammoniumionen und von Formiationen zugeführt. Die Konzentration des Substrats soll 50 bis 100 % der maximal möglichen Konzentration betragen, darf aber nicht über 2 000 mmol/l, vorzugsweise nicht über 1 000 mmol/l, sein. Die Konzentration an Ammoniumionen muss für einen vollständigen Umsatz des Substrats der Substratmenge äquimolar sein, auch ein Überschuss an Ammoniumionen stört jedoch die Umsetzung nicht. Andererseits aber kann wegen der beim erfindungsgemässen Verfahren zwangsläufig eintretenden Rückvermischung zwischen Produkt und Substrat vor der Membran ein quantitativer Umsatz ohnehin nicht erreicht werden. In vielen Fällen genügt es daher, wenn die Ammoniumionen in einem gewissen Unterschuss, beispielsweise nur mit etwa 80 % der für einen quantitativen Umsatz des Substrats erforderlichen Menge, eingesetzt werden. Die Konzentration an Formiationen liegt zwischen 100 und 6 000 mmol/l, vorzugsweise zwischen 300 und 2 000 mmol/l. Als Formiate werden bevorzugt Natrium- oder Kaliumformiat verwendet. Besonders vorteilhaft ist es jedoch, wenn man der zuzuführenden Substratlösung die Ammoniumionen und die Formiationen zusammen in Form von Ammoniumformiat zusetzt.

Während der Umsetzung muss über die Membran ein Differenzdruck von 0,1 bis 15 bar, vorzugsweise von 0,2 bis 3 bar, aufrechterhalten werden, was durch Verwendung einer entsprechend dimensionierten Dosierpumpe für die zuzuführende Substratlösung und gegebenenfalls durch ein Drosselventil im Filtratstrom hinter der Membran erreicht wird. Der Differenzdruck bewirkt, dass ein Filtratstrom mit der gewünschten Geschwindigkeit durch die Membran hindurchtritt. Der Absolutdruck auf der Druckseite der Membran sollte zweckmässigerweise so eingestellt werden, dass auch bei kräftigem Rühren oder Umpumpen im Reaktionsraum vor der Membran zur Erzeugung einer kräftigen Turbulenz längs der Membran und damit zur Vermeidung einer Belegung der Membran mit den Enzymen oder dem im Molekulargewicht vergrösserten Coenzym an keiner Stelle der Druck so weit abgesenkt wird, dass es zu einem Entgasen des Reaktionsgemisches auf der Druckseite kommt. Der Membranreaktor wird auf einer für enzymatische Umsetzungen üblichen Temperatur zwischen 25 und 50 °C gehalten. Desgleichen wird der pH des Reaktionsgemisches während der Umsetzung in dem für enzymatische Umsetzungen üblichen Bereich zwischen 5 und 9 gehalten.

Für die Durchführung des erfindungsgemässen Verfahrens geeignete Formiatdehydrogenasen können beispielsweise aus Candida boidinii oder aus Pseudomonas oxalaticus isoliert werden. Ein Beispiel für eine beim erfindungsgemässen Verfahren verwendbare substratspezifische Dehydrogenase ist die vielfältig einsetzbare L-Alanindehydrogenase, die sich aus Bacillus subtilis gewinnen lässt. Mit ihrer Hilfe kann beispielsweise Brenztraubensäure in L-Alanin, 2-Oxo-4-methylvaleriansäure in L-Leucin, 2-Oxo-3-methylvaleriansäure in L-Isoleucin, 2-Oxo-3-methylbuttersäure in L-Valin oder 2-Oxovaleriansäure in L-Norvalin umgewandelt werden. Zweckmässigerweise werden die umzusetzenden α-Ketocarbonsäuren in Form ihrer Natrium- oder Kaliumsalze eingesetzt. Da bei der Umwandlung von α-Ketocarbonsäuren in die entsprechenden Aminosäuren ein optisch aktives Zentrum neu gebildet wird, lässt sich die Produktkonzentration im Filtratstrom mit Hilfe eines Polarimeters kontinuierlich messen. Die gebildete Aminosäure kann aus dem Filtrat in an sich bekannter Weise, z. B. mit

Hilfe eines sauren Ionenaustauschers, gewonnen werden.

Im nachfolgenden Beispiel wird das erfindungsgemässe Verfahren anhand der Umwandlung von Natriumpyruvat in L-Alanin näher erläutert :

Beispiel 1

Ein auf einer Temperatur von 40 °C gehaltener Flach membranreaktor mit einem Volumen von 10 ml, der mit einem Magnetrührer und einer Ultrafiltrationsmembran von 62 mm Durchmesser mit einer nominellen Ausschlussgrenze von 5 000 (Lieferfirma : Amicon, Witten ; Typ DM 5) ausgestattet war, wurde zur Sterilisation mittels einer auf eine Fördergeschwindigkeit von 20 ml/Stunde eingestellten Dosierpumpe ca. 5 Stunden lang mit wässriger Formaldehydlösung durchgespült. Anschliessend wurde während weiterer ca. 5 Stunden die Formaldehydlösung durch destilliertes Wasser verdrängt. Danach wurde, ebenfalls mit einer Fördergeschwindigkeit von 20 ml/Stunde, ca. 2,5 Stunden lang eine über ein Sterilfilter (0,2 μm) filtrierte Substratlösung zugeführt, die 500 mmol/l Natriumpyruvat, 400 mmol/l Ammoniumformiat und 50 mmol/l Natriumdihydrogenphosphat enthielt und mit Natronlauge auf pH 8 eingestellt war. Darauf wurden anstelle der Substratlösung 10 ml einer Coenzymlösung zudosiert, die 3,66 mmol/l NADH, gebunden an ein Polyoxyäthylen mit einem mittleren Molekulargewicht von 10 000, und 50 mmol/l eines Phosphatpuffers für pH 8 enthielt. Nach der vollständigen Zugabe der Coenzymlösung wurde weiter die obige Substratlösung mit einer Fördergeschwindigkeit von 20 ml/Stunde zugeführt. Dann wurden dem Reaktionsraum vor der Membran durch eine seitliche Bohrung 100 mg Formiathydrogenase (Aktivität 6,74 μmol/mg · Minute bei Formiat als Substrat, 40 °C und pH 8) in Form einer wässrigen Glycerinlösung (50 Gewichtsprozent Glycerin ; 10 mg Formiatdehydrogenase/ml) und 1,62 mg L-Alanindehydrogenase (Aktivität 415 μmol/mg · Minute bei Pyruvat als Substrat, 40 °C und pH 8) in Form einer wässrigen Ammoniumsulfatlösung (2,4 mol/l (NH$_4$)$_2$ SO$_4$ ; 5 mg L-Alanindehydrogenase/ml) mittels einer Spritze zugesetzt. Bei der gewählten Einstellung betrug das Verhältnis der Aktivitäten von Formiatdehydrogenase und L-Alanindehydrogenase 1 : 1. Der Umsatz wurde kontinuierlich mit Hilfe einer in den Filtratstrom eingebauten Polarimeter-Durchflussküvette verfolgt. Der Differenzdruck über die Membran betrug zu Beginn 0,2 bar, stieg allmählich auf 0,5 bar an und blieb dann konstant. Innerhalb einer Betriebszeit von insgesamt ca. 50 Stunden wurden 175 mmol L-Alanin erhalten. Die maximale Umsatzrate betrug 6,45 mmol L-Alanin/Stunde.

Die Ausbeute betrug 108 mmol L-Alanin, bezogen auf 1 mg L-Alanindehydrogenase, bzw. 1,75 mmol L-Alanin, bezogen auf 1 mg Formiatdehydrogenase.

Beispiel 2

Die Sterilisierung des 10 ml-Flachmembranreaktors (Membran mit einer nominellen Ausschlußgrenze von 5 000 ; Lieferfirma : Amicon, Witten ; Type YM 5), der auf einer Temperatur von 25 °C gehalten wurde, erfolgte wie im Beispiel 1.

Mit einer Fördergeschwindigkeit von 20 ml/Stunde wurde dann ca. 2,5 Stunden lang eine steril filtrierte Lösung zugeführt, die 400 mmol/l Natriumpyruvat, 400 mmol/l Ammoniumformiat und 50 mmol/l Natriumdihydrogenphosphat enthielt und mit Natronlauge auf pH 8 eingestellt war. Anschließend wurde anstelle der Substratlösung mit einer Fördergeschwindigkeit von 4 ml/Stunde eine Mischung aus 7 ml der Coenzymlösung gemäß Beispiel 1 und aus 138 mg Formiatdehydrogenase (Aktivität 0,85 μmol/mg · Minute bei Formiat als Substrat, 25 °C und pH 8) in Form einer wäßrigen Glycerinlösung (50 Gewichtsprozent Glycerin ; 10 mg Formiatdehydrogenase/ml) zugeführt. Dann wurden dem Reaktionsraum durch eine seitliche Bohrung 2,78 mg L-Alanindehydrogenase (Aktivität 233 μmol/mg · Minute bei Pyruvat als Substrat, 25 °C und pH 8) in Form einer wäßrigen Ammoniumsulfatlösung (2,4 mol/l (NH$_4$)$_2$SO$_4$ ; 5 mg L-Alanindehydrogenase/ml) mittels einer Spritze zugesetzt. Das Verhältnis der Aktivitäten von Formiatdehydrogenase und L-Alanindehydrogenase betrug sornit 1 : 4. Innerhalb einer Betriebszeit von 59 Stunden wurden 48 mmol L-Alanin erhalten, entsprechend einer Ausbeute von 17,3 mmol L-Alanin, bezogen auf 1 mg L-Alanindehydrogenase, bzw. 0,35 mmol L-Alanin, bezogen auf 1 mg Formiatdehydrogenase.

Beispiel 3

Die Sterilisierung des 10 ml-Flachmembranreaktors (Membran mit einer nominellen Ausschlußgrenze von 5 000 ; Lieferfirma : Amicon, Witten ; Type YM 5), der auf einer Temperatur von 25 °C gehalten wurde, erfolgte wie im Beispiel 1.

Mit einer Fördergeschwindigkeit von 20 ml/Stunde wurde dann ca. 2,5 Stunden lang eine steril filtrierte Lösung zugeführt, die 400 mmol/l Natriumpyruvat, 800 mmol/l Ammoniumformiat und 50 mmol/l Natriumdihydrogenphosphat enthielt und mit Natronlauge auf pH 8 eingestellt war. Anschließend wurde anstelle der Substratlösung mit einer Fördergeschwindigkeit von 4 ml/Stunde eine Mischung aus 10 ml der Coenzymlösung gemäß Beispiel 1 und aus 85 mg Formiatdehydrogenase (Aktivität 0,85 μmol/mg · Minute bei Formiat als Substrat, 25 °C und pH 8) in Form einer wässrigen Glycerinlösung (50 Gewichtsprozent Glycerin ; 10 mg Formiatdehydrogenase/ml) zugeführt. Dann wurden dem Reaktionsraum durch eine seitliche Bohrung 0,94 mg L-Alanindehydrogenase (Aktivität 233 μmol/mg · Minute bei Pyruvat als Substrat, 25 °C und pH 8) in Form einer wässrigen Ammoniumsulfatlösung (2,4 mol/l (NH$_4$)$_2$SO$_4$ ; 5 mg L-Alanindehydrogenase/ml) mittels einer Spritze zugesetzt. Das Verhältnis der Aktivitäten betrug somit 1 : 2,2. Innerhalb einer Betriebszeit von 140 Stunden wurden 92 mmol L-Alanin, erhalten, entsprechend einer Ausbeute von 98 mmol L-Alanin, bezogen auf 1 mg L-Alanindehydrogenase, bzw. 1,08 mmol L-Alanin, bezogen auf 1 mg Formiatdehydrogenase.

### Ansprüche

1. Verfahren zur kontinuierlichen enzymatischen Umwandlung von wasserlöslichen $\alpha$-Ketocarbonsäuren in die entsprechenden Aminosäuren in Gegenwart einer substratspezifischen Dehydrogenase, von Ammoniumionen und von durch Bindung an ein wasserlösliches Polymeres im Molekulargewicht vergrössertem Nicotinamidadenin-dinucleotid ($NAD^+$/NADH) in einem mit einer Ultrafiltrationsmembran ausgestatteten Membranreaktor unter gleichzeitiger Regenerierung von NADH aus $NAD^+$ mittels Formiationen in Gegenwart einer Formiatdehydrogenase, dadurch gekennzeichnet, dass man einem Membranreaktor, dessen Membran einen mittleren Porendurchmesser von 1 bis 3 nm aufweist, und der eine Lösung der Formiatdehydrogenase, der substratspezifischen Dehydrogenase und von 0,1 bis 10 mmol/l an ein Polyoxyäthylen mit einem durchschnittlichen Molekulargewicht zwischen 500 und 50 000 gebunden vorliegendem $NAD^+$/ NADH enthält, kontinuierlich eine wässrige Lösung von 50 bis 100 % der maximal löslichen Menge, jedoch nicht über 2 000 mmol/l, der umzusetzenden $\alpha$-Ketocarbonsäure in Form eines wasserlöslichen Salzes als Substrat, einer der Substratmenge etwa äquimolaren Menge an Ammoniumionen, und von 100 bis 6 000 mmol/l eines Formiats zuführt, über die Membran einen Differenzdruck von 0,1 bis 15 bar aufrechterhält und hinter der Membran kontinuierlich einen die gebildete Aminosäure enthaltenden Filtratstrom abführt.

2. Verfahren nach Anspruch 1, dadurch gekennzeichnet, dass man die Formiatdehydrogenase und die substratspezifische Dehydrogenase in solchen Mengen einsetzt, dass das Verhältnis ihrer Aktivitäten zwischen 1 : 1 und 1 : 5 liegt.

3. Verfahren nach einem der Ansprüche 1 oder 2, dadurch gekennzeichnet, dass man der zuzuführenden Substratlösung die Ammoniumionen und die Formiationen zusammen in Form von Ammoniumformiat zusetzt.

### Claims

1. A process for the continuous enzymatic conversion of water-soluble $\alpha$-keto carboxylic acids into the corresponding amino acids in the presence of a substrate-specific dehydrogenase, ammonium ions and nicotinamide-adenine-dinucleotide ($NAD^+$/NADH) increased in molecular weight by binding to a water-soluble polymer, in a membrane reactor equipped with an ultrafiltration membrane, with the simultaneous regeneration of NADH from $NAD^+$ by formate ions in the presence of a formate dehydrogenase, characterised in that an aqueous solution of from 50 to 100 % of the saturation concentration, but not more than 2 000 mmol/l of the $\alpha$-keto carboxylic acid to be converted, in the form of a water-soluble salt as substrate, a quantity of ammonium ions which is approximately equimolar to the quantity of substrate, and from 100 to 6 000 mmol/l of a formate are continuously delivered to a membrane reactor, the membrane of which has an average pore diameter of from 1 to 3 nm, and which contains a solution of the formate dehydrogenase, the substrate-specific dehydrogenase and from 0.1 to 10 mmol/l of $NAD^+$/NADH bound to a polyoxyethylene having an average molecular weight of from 500 to 50 000, a differential pressure of from 0.1 to 15 bars is maintained over the membrane and a filtrate flow containing the amino acid which has formed is continuously removed downstream of the membrane.

2. A process according to claim 1, characterised in that the formate dehydrogenase and the substrate-specific dehydrogenase are used in quantities such that the ratio of their activities is from 1 : 1 to 1 : 5.

3. A process according to one of claims 1 or 2, characterised in that the ammonium ions and the formate ions are added together in the form of ammonium formate to the substrate solution to be delivered.

### Revendications

1. Procédé pour la conversion enzymatique en continu d'acides $\alpha$-céto-carboxyliques solubles dans l'eau en les acides aminés correspondants, en présence d'une déshydrogénase spécifique au substrat, d'ions ammonium et de nicotinamide-adénine-dinucléotide ($NAD^+$/NADH) à poids moléculaire accru par liaison avec un polymère hydrosoluble, dans un réacteur à membrane pourvu d'une membrane d'ultra-filtration, avec régénération simultanée d'NADH provenant du $NAD^+$, au moyen d'ions formiates en présence d'un formiate déshydrogénase, procédé caractérisé en ce que, dans un réacteur à membrane dont la membrane présente un diamètre de pores de 1 à 3 nm et qui contient une solution du formiate déshydrogénase, de la déshydrogénase spécifique au substrat et $NAD^+$/NADH sous forme liée à un polyoxyéthylène à poids moléculaire moyen compris entre 500 et 50 000, on amène en continu une solution aqueuse de 50 à 100 % de la quantité maximale soluble, mais n'excèdent pas 2 000 millimoles par litre, de l'acide $\alpha$-céto-carboxylique à convertir sous la forme d'un sel hydrosoluble comme substrat, une quantité d'ions d'ammonium sensiblement équimolaire à la quantité de substrat et de 100 à 6 000 millimoles par litre d'un formiate, une pression différentielle de 0,1 à 15 bars étant maintenue au-dessus de la membrane, et le courant de filtrat qui contient l'acide aminé formé étant extrait en continu derrière la membrane.

2. Procédé suivant la revendication 1, caractérisé en ce que l'on met en œuvre le formiate déhydrogénase et la déhydrogénase spécifique au substrat dans des quantités telles que le rapport entre leurs activités se situe entre 1/1 et 1/5.

3. Procédé suivant l'une des revendications 1 ou 2, caractérisé en ce que l'on ajoute à la solution de substrat à amener en continu les ions ammonium et les ions formiate sous la forme de formiate d'ammonium.